# EUROPEAN PATENT APPLICATION

(11) **EP 3 708 066 A1**
(43) Date of publication of application: **16.09.2020**
(21) Application number: 19161943.6
(22) Date of filing: 11.03.2019
(51) Int. Cl.: A61B 5/00, A61B 5/01, A61B 5/055, A61B 5/107, A61B 8/00

(54) **MEDICAL DEVICE**

(71) Applicant: Caressoma AG, 8005 Zürich (CH)
(72) Inventor: BOROWKA, Sophia, 8004 Zürich (CH)
(74) Representative: Frenkel, Matthias Alexander

(57) **Abstract**

The present invention generally relates to medical devices and more particularly to medical imaging. More specifically, the present invention relates to a medical device, a related method and a medical system comprising the medical device. An embodiment of the medical device comprises at least one measuring layer and a processing layer. The at least one measuring layer comprises: one or more thermographic sensing units configured to detect infrared radiation emitted by a portion of a body of a user, and one or more additional medical imaging units configured to acquire additional imaging information about the portion of the body. The processing layer comprises one or more processing units configured to process the detected infrared radiation and the acquired additional imaging information to enable reconstruction of a combined medical image based on the detected infrared radiation and the acquired additional imaging information.

## Description

The present invention generally relates to medical devices and more particularly to medical imaging. More specifically, the present invention relates to a medical device, a related method and a medical system comprising the medical device.

Physical exercise can lead to injuries, in particular of muscles, ligaments and the like. It is desirable to detect such injuries as soon as possible in a non-invasive way. Nowadays, non-invasive imaging techniques are either not suitable to quickly detect such injuries, are hard to interpret or are not accurate enough.

For example, locating an inflamed area can usually only be done reliably with a magnetic resonance imaging (MRI) system. However, MRI is time- and cost-intensive, plus the availability is low due to high acquisition costs of MRI systems. While pain in the case of an inflammatory response can be mentioned by the patient, it can spread further than where the actual inflammation is, which makes a diagnosis difficult without any imaging technology. In principle, it is possible to use thermography in order to track the inflammatory response. Doctors have applied thermography in different areas of application (in particular with respect to tumor related issues) but the information is not considered of being clinically relevant enough.

As another example, resolving muscle cracks/tears of single muscle fibrils requires an accuracy of micrometer precision which can only be achieved with high frequency ultrasound. However, high frequency ultrasound has a low penetration depth. Nowadays, the penetration depth of high frequency ultrasound devices is not sufficient to display such injuries.

Even bigger than micrometer-sized muscle cracks are hard to read off, even though the technology for this resolution exists on the market, given the ultrasound picture needs to be interpreted in the right way. So there are problems in the interpretation of the resulting picture/data. A long training is necessary to perform this task intuitively. Ultrasound images can only be interpreted by a several-years trained professional and are limited in their information quality. Diagnostic imaging is therefore - in practice - not accessible to physiotherapists or personal trainers, who are, however, often closest to the affected patient or client. This means that they have to rely on their intuition, which has to be trained for years to improve. This results in a loss of accuracy of the treatment, increased treatment time, reduced patient adherence rate and higher costs for the health care system. In short, reading ultrasound data needs expert knowledge and years of training.

In order to overcome deficiencies of single imaging techniques, hybrid imaging techniques and related methods have been proposed combining at least two different measuring techniques. Such techniques usually relate to a combination of computer tomography (CT) using x-rays and MRI. The big downside of CT is the undesirable radiation exposure, while the disadvantages of MRI are its high costs, non-portability and the still often necessary use of toxic contrast agents to improve the image resolution. However, the resolution of CT (bone structure) and MRI (soft tissue) are unparalleled.

These traditional techniques have recently been combined with artificial intelligence (e.g. via image feature extraction that can be automated by/with the help of neural networks) to get augmented results and help evade invasive diagnostic methods (e.g. biopsies). However, systems using hybrid imaging techniques based on a combination of CT and MRI are non-portable and expose the patient to radiation and/or potentially to contrast agents.

There is a need for a medical device, a related method and a medical system comprising such a medical device exposing the patient to low or even no radiation and still enabling the reconstruction of medical images with sufficient and reliable information.

According to a first aspect of the present invention, a medical device is provided. The medical device comprises at least one measuring layer and a processing layer. The at least one measuring layer comprises one or more thermographic sensing units and one or more additional medical imaging units. The one or more thermographic sensing units are configured to detect infrared radiation emitted by a portion of a body of a user. The one or more additional medical imaging units are configured to acquire additional imaging information about the portion of the body. The processing layer comprises one or more processing units. The processing units are configured to process the detected infrared radiation and the acquired additional imaging information to enable reconstruction of a combined medical image based on the detected infrared radiation and the acquired additional imaging information.

Alternatively, the one or more thermographic sensing units may be configured to detect infrared radiation emitted by a probe, in general. The one or more additional medical imaging units may be configured to acquire additional imaging information about the probe. The probe may also be referred to as a probe to be examined or a probe under examination.

The body may be a human or an animal body. The portion of the body may comprise or be or be part of a body part. The portion of the body or the body part may include or be or be part of an arm, a leg or another portion of the body. The medical device may be or may be configured as a portable medical device or a portable medical imaging device.

Portable may be understood as being able to be easily carried or moved, in particular because being of a lighter and/or smaller version than usual. If portable, the medical device may be referred to as a portable medical device or portable medical imaging device (if additionally having a display functionality), or portable medical (imaging) device when related to both a device with or without display functionality, e.g. with or without a display. The portable medical (imaging) device may thus be able to be easily carried or moved, in particular because the portable medical (imaging) device may be lighter and/or smaller than medical imaging devices available today. For example, the portable medical (imaging) device may be carried or moved with one hand. The portable medical (imaging) device may have a size similar to a book, a magazine, a laptop, a tablet PC or the like. The portable medical (imaging) device may even be worn by a user and may thus be referred to as wearable medical imaging device. In this case, the one or more thermographic sensing units may detect infrared radiation emitted by a portion of a body of a user by which it is worn and the one or more additional medical imaging units may acquire additional imaging information about the portion of the body of the user by which it is worn, while the wearable medical imaging device is worn by the user.

The one or more additional imaging units may respectively comprise a sensing or detecting unit only without any emitting and transmitting unit. In this case, one or more additional emitting or transmitting units may be provided. Alternatively, the one or more additional imaging units may comprise or be configured as one or more sensing or detecting units and one or more emitting or transmitting units. The one or more emitting or transmitting units may be configured to emit or transmit respective signals. The one or more sensing or detecting units may be configured to sense or detect the respective signals after at least partial reflection on the portion of the body or the probe.

Thermography may comprise contact or non-contact thermography. Thermography may be carried out to detect short- to long-wavelength infrared light. In the latter, the one or more thermographic sensing units may comprise or be configured as one or more thermographic cameras. In the former case, the one or more thermographic sensing units may comprise or be configured as one or more liquid crystal polymer (LCP) foils. Alternatively to the LCP foils, thermistors may be used. Both sensing unit implementation examples (the LCP foils and the thermistors) can be used in contact thermography. In case of the thermistors, an array of thermistors might be used to achieve a two-dimensional thermographic image. Thermistors are extremely accurate, even though a measurement can take up to 10 seconds until it is finished.

The one or more processing units may be configured to process the detected infrared radiation and the acquired additional imaging information to reconstruct the combined medical image based on the detected infrared radiation and the acquired additional imaging information in the medical device, e.g. the portable medical (imaging) device. Alternatively, the one or more processing units may be configured to pre-process the detected infrared radiation and the acquired additional imaging information to enable another entity or unit, e.g. an entity external to the medical device, for example the portable medical (imaging) device, to reconstruct the combined medical image based on the pre-processed detected infrared radiation and the pre-processed acquired additional imaging information.

The one or more thermographic sensing units may be arranged in a first plane in a depth direction of the medical device, e.g. the portable medical (imaging) device. The one or more additional medical imaging units may be arranged in a second plane in the depth direction of the medical device, e.g. the portable medical (imaging) device. The first plane may be the same as the second plane. Alternatively, the first plane may be different from the second plane. The depth direction may be the direction of the shortest extension of the medical device, e.g. the portable medical (imaging) device, i.e. the depth direction may have a smaller extension than the extension of the medical device, e.g. the portable medical (imaging) device, in its height and width direction. The depth direction of the medical device, e.g. the portable medical (imaging) device, may correspond to the direction of a normal on the (biggest) surface of the measuring layer. In other words, the depth direction of the medical device, e.g. the portable medical (imaging) device, may correspond to the direction in which the one or more thermographic sensing units and/or the one or more additional imaging units are directed.

The at least one measuring layer may comprise or be configured as multiple measuring layers. In this case, the one or more thermographic sensing units may be arranged in a first measuring layer of the multiple measuring layers. Further, the one or more additional medical imaging units may be arranged in a second measuring layer of the multiple measuring layers. The first measuring layer may be the layer which, when the portable imaging device is used, is closer to or in contact with the body of the user (i.e. the probe) than the second measuring layer. The one or more thermographic sensing units arranged in the first measuring layer may be arranged to overlap, in the height and/or width direction of the medical device, e.g. the portable medical (imaging) device, with the one or more additional medical imaging units arranged in the second measuring layer. In this case, the number of thermographic sensing units and the number of additional medical imaging units can be optimised. Alternatively or additionally, the one or more thermographic sensing units arranged in the first measuring layer may be arranged to not overlap, in the height and/or width direction of the medical device, e.g. the portable medical (imaging) device, with the one or more additional medical imaging units arranged in the second measuring layer. In this case, the measurement accuracy can be optimised as any influences, e.g. distortions influencing the measurement accuracy, between the one or more thermographic sensing and the one or more additional medical imaging units can be reduced.

The at least one measuring layer may be bendable. Even though the accuracy of a temperature measurement using a thermographic camera is theoretically high, the actual accuracy of the measurement highly depends on surrounding interference factors and distance to the probe to be measured. Thus, to increase the accuracy of the measurement, the distance to the probe might be maintained as stable and as accurate as possible. This can be ensured or at least improved by a bendable at least one measurement layer. By bending the measurement layer in a way similar to the shape of the portion or probe to be examined, any of the sensing units, e.g. the one or more thermographic sensing units and/or the one or more additional medical imaging units, may have the same or at least a similar distance to the portion or probe under examination. In other words, the bendable measuring layer may have a constant distance to the body or probe to be examined / under examination throughout its measuring surface.

The one or more thermographic sensing units may comprise or be configured as one or more cooled infrared detectors. This may increase accuracy. Alternatively or additionally, the one or more thermographic sensing units may comprise or be configured as one or more uncooled infrared detectors. The actual accuracy can therefore be about +- 2°C. Contact thermography may be used to increase resolution. Alternatively or additionally, non-contact thermography may be used. Contact thermography requires direct body contact to be accurate. Direct contact may be ensured by using a bendable measurement layer as described above.

At least one or more portions of the at least one measuring layer may be movable. For example, the complete at least one measuring layer or a subset of the at least one measuring layer may be movable. In case the at least one measuring layer comprises or is configured as a first measuring layer and a second measuring layer, at least portions of the first measuring layer and/or at least portions of the second measuring layer me be movable. When moving for example the first measuring layer, the second measuring layer can carry out measurements without any or at least with reduced distortions or influences potentially caused by the first measuring layer. In this way, measurement accuracy can be improved.

The at least one measuring layer may comprise a plurality of measuring sections. Each of the plurality of measuring sections may comprise at least one of the one or more thermographic sensing units and/or at least one of the one or more additional medical imaging units. The measuring sections may respectively be separated from each other by one or more slits. In this way, the at least one measurement layer can be configured to be bendable. The slits may facilitate the bending of the at least one measurement layer. Accuracy of the measurement can thus be increased as the distance of the at least one measuring layer to the probe may be maintained as stable as possible. One or more of the plurality of measuring sections may be moveable.

The processing layer may further comprise one or more control units. The one or more control units may be configured to control the one or more thermographic sensing units. The one or more control units may be configured to control the one or more additional medical imaging units. For example, the one or more control units may be configured to control the one or more thermographic sensing units to detect the infrared radiation at a first period of time. The one or more control units may be configured to control the one or more additional medical imaging units to acquire the additional imaging information at a second period of time. The first period of time and the second period of time may at least partly/partially overlap each other. For example, the first period of time and the second period of time may substantially coincide with each other. In a specific implementation, the first period of time may be a first point of time and the second period of time may be a second point of time. The first point of time may at least substantially correspond to the second point of time.

The medical device, e.g. the portable medical (imaging) device, may further comprise a buffer layer between the at least one measuring layer and the processing layer. The buffer layer may be bendable. The buffer layer may aid in the measurement of how much the at least one measuring layer is bent/curved and where it is curved/bent. The bending can be measured in various ways. One example is the usage of accelerometers. Accelerometers are relatively cheap and detect changes in position. The accelerometers may be attached to e.g. both ends of the buffer layer. The ends of the buffer layer may be defined as one end being closest to the processing layer and the other end being closest to the measuring layer. If the accelerometers are attached to both ends of the buffer layer, their differential allows for an accurate positioning of the end of the buffer layer that is connected to the measuring layer. In this way, the bending or amount of bending of the measurement layer may be derived or determined. One other example is the use of optical fibres. The optical fibers may be attached or enclosed in a stiffer bendable metal tube. The fibre's or fibres' bending radius may be computed from the increase in signal dampening. In this way, the bending or amount of bending of the measurement layer may be derived or determined.

The medical device, e.g. the portable medical (imaging) device, may further comprise a mounting unit. The mounting unit may be configured to mount, attach or fix the portable medical imaging device to the body of the user. By mounting the medical device, e.g. the portable medical (imaging) device to the body of a user, the medical device, e.g. the portable medical (imaging) device, can be worn, e.g. during exercise, and can carry out measurements while being worn. The mounting unit can be realised in various ways. If used as a wearable device, there are several implementations for a mounting. The mounting unit may include or be configured as a combination of straps. The straps may comprise or be made of an easy-to-clean material (e.g. elastic rubber-based band). The straps may, when mounted, enclose the portion of the body or body part in question (to be examined). The mounting unit may include or be configured as a combination of temporarily-adhesive foam cushions. The cushions may temporarily be attached to the portion of the body or the body part to be examined.

The medical device, e.g. the portable medical (imaging) device, may further comprise a holding section. The holding section may be configured to hold a display device, a tablet PC or a smartphone. The size of the holding section may be adjustable to hold different devices. For example, a reconstructed medical image obtained from the detected infrared radiation and the acquired additional imaging information can be displayed on a display or device held by the holding section. In case the medical device includes or holds a display or a display device in its holding section the medical device may be referred to as a medical imaging device.

The medical device, e.g. the portable medical (imaging) device, may further comprise a communication interface for communicating with one or more server units. The medical device, e.g. the portable medical (imaging) device, may transmit the (pre-)processed detected infrared radiation and the (pre-)processed acquired additional imaging information to the one or more server units. The interface may enable wireless communication with the one or more server units.

The one or more processing units may be configured to reconstruct the combined medical image based on the detected infrared radiation and the acquired additional imaging information. Alternatively or additionally, the one or more processing units may be configured to analyse the reconstructed combined medical image for elevated temperature in the portion of the body. From the elevated temperature, areas of inflammation may be derived. From the areas of inflammation and potentially additional information, injuries or other abnormalities in the portion of the body may be determined. In other words, the one or more processing units may be configured to analyse the reconstructed combined medical image for elevated temperature, inflammations, injuries and other abnormalities or diseases related to inflammation in the portion of the body.

According to a second aspect of the present invention, a medical system is provided. The medical system comprises the portable imaging device according to the first aspect as described herein and one or more server units. The one or more server units are configured to reconstruct the combined medical image based on the detected infrared radiation and the acquired additional imaging information. Alternatively or additionally, the one or more server units are configured to analyse the reconstructed combined medical image for elevated temperature, inflammations, injuries and other abnormalities or diseases related to inflammation in the portion of the body. Alternatively or additionally, the one or more server units are configured to store a plurality of images of various portions of the body in healthy and unhealthy states. Alternatively or additionally, the one or more server units are configured to provide one or more of a plurality of stored images of the portion of the body to the medical device, e.g. the portable medical (imaging) device.

For example, the medical device, e.g. the portable medical (imaging), device may transmit the (pre-)processed detected infrared radiation and the (pre-)processed acquired additional imaging information to the one or more server units. The one or more server units may be configured to process the (pre-)processed detected infrared radiation and the (pre-)processed acquired additional imaging information further and to thereby reconstruct the combined medical image from the detected infrared radiation and the acquired additional imaging information.

According to a third aspect of the present invention, a method for monitoring a portion of a body of a user is provided. The method comprises detecting infrared radiation emitted by the portion of the body. The method further comprises acquiring additional imaging information about the portion of the body. The method further comprises processing the detected infrared radiation and the acquired additional imaging information to enable reconstruction of a combined medical image based on the detected infrared radiation and the acquired additional imaging information.

The method may further comprise reconstructing the combined medical image based on the processed detected infrared radiation and the processed acquired additional imaging information.

The method may at least in part be performed in the medical device, e.g. the portable medical (imaging) device, according to the first aspect as described herein.

The one or more additional medical imaging units and specific exemplary implementation details thereof will now be described in more detail. These details can equally be applied to the medical device, e.g. the portable medical (imaging) device, according to the first aspect, to the medical system according to the second aspect as well as to the method according to the third aspect.

The one or more additional medical imaging units may comprise or be configured as one or more ultrasound imaging units. Alternatively or additionally, the one or more additional medical imaging units may comprise or be configured as one or more thermoacoustic imaging (TAT) units. Alternatively or additionally, the one or more additional medical imaging units may comprise or be configured as one or more x-ray or gamma-ray radiography imaging units. Alternatively or additionally, the one or more additional medical imaging units may comprise or be configured as one or more magnetometric imaging units. Alternatively or additionally, the one or more additional medical imaging units may comprise or be configured as one or more elastographic imaging units. Alternatively or additionally, the one or more additional medical imaging units may comprise or be configured as one or more ultrasound elastography imaging units. Alternatively or additionally, the one or more additional medical imaging units may comprise or be configured as one or more electrographic imaging units. Alternatively or additionally, the one or more additional medical imaging units may comprise or be configured as one or more photoacustic/optoacoustic imaging (PAT) units. Alternatively or additionally, the one or more additional medical imaging units may comprise or be configured as one or more magnetometric imaging units. Alternatively or additionally, the one or more additional medical imaging units may comprise or be configured as one or more electrical impedance measurement (with or without additional transmitting/external stimulation units) imaging units. Alternatively or additionally, the one or more additional medical imaging units may comprise or be configured as one or more magnetic resonance imaging units. Alternatively or additionally, the one or more additional medical imaging units may comprise or be configured as one or more functional magnetic resonance imaging units. Alternatively or additionally, the one or more additional medical imaging units may comprise or be configured as one or more tactile imaging/mechanical imaging units. Alternatively or additionally, the one or more additional medical imaging units may comprise or be configured as one or more medical optical imaging units.

The one or more medical optical imaging units may be or comprise one or more laser light based imaging units. Alternatively or additionally, the one or more additional medical imaging units may comprise or be configured as one or more medical optical imaging units of a same or different type which can but do not necessarily have to include laser light.

The one or more elastographic imaging units may be one or more ultrasound elastography units or magnetic resonance elastography (MRE) units, for example.

The one or more ultrasound elastography units may be one or more quasistatic elastography/strain imaging units, acoustic radiation force impulse imaging (ARFI) units, Shear-wave elasticity imaging (SWEI) units, supersonic shear imaging (SSI) units, transient elastography units or time-resolved pulse elastography units, for example.

The one or more tactile imaging/mechanical imaging units may be one or more laser vibrometry units, pressure sensor units or ultrasound imaging units, for example.

The one or more electrographic imaging units may be one or more electromyography (EMG) units, electroneuronography or electroneurography (ENoG) units, nerve conduction studies (NCS) units, electrocardiography (ECG or EKG) units, electroencephalography (EEG) units, Electrocorticography or intracranial EEG (iEEG or ECoG) units, Electrooculography (EOG) units, Electroolfactography (EOG) units, Electroretinography (ERG) units, Electronystagmography (ENG) units, Electrocochleography (ECOG) units, Electroantennography (EAG) units, Electrogastrography (EGG) units, Electrogastroenterogram (EGEG) units, Electroglottography (EGG) units or Electropalatography (EPG) units, for example.

The one or more x-ray or gamma-ray radiography imaging units may be one or more projectional radiography units, computed tomography (CT) units, computed axial tomography (CAT) units, dual energy x-ray absorptiometry (DEXA) units, fluoroscopy units, angiography units, contrast radiography units, positron emission tomography (PET) units or single-photon emission computed tomography (SPECT) units, for example.

The one or more magnetometric imaging units may be one or more magnetoencephalography (MEG) units, for example.

The one or more medical optical imaging units may be one or more diffuse optical imaging units, near-infrared spectroscopy (NIRS) units, pulse oximetry units, photoplethysmography units, functional neuroimaging units, functional near infrared spectroscopy (fNIRS) units or optical coherence tomography units, for example.

The one or more additional medical imaging units may comprise or be configured as one or more ultrasound imaging units. In this way, ultrasound measurements can be carried out. Ultrasound is a non-invasive, non-toxic medical imaging technology and its radiation is not harmful for the body. On the contrary, it is successfully used for therapeutic treatments as well, in particular in the rehabilitative care and beauty industry. The measurement accuracy reached varies with the body penetration depth. In general, the deeper the penetration, the lower the resolution. Ultrasound technology allows for a broad range of measurements in A-mode, B-mode, C-mode, M-mode, Doppler mode, Pulse inversion mode, Harmonic mode, or to perform an elastography, myography, among others. The costs for ultrasound devices are expected to be decreased given that a new technology has entered the market using capacitive micromachined ultrasonic transducers (CMUTs) instead of the traditional and highly expensive transducers based on piezoelectricity. In case the additional medical imaging units comprise or configured as one or more ultrasound imaging units, the one or more ultrasound imaging units may comprise or be configured as one or more CMUTs.

The additional medical imaging units may comprise or be configured as one or more medical optical imaging units, e.g. one or more laser light based imaging units. In this way, optical measurements can be carried out. For example, light from the visible spectrum (380-740 nm) to the near-infrared spectrum (940 nm) can be used and detected. A fixed-frequency light may be emitted onto the probe/body by one or more transmitting units of the one or more additional medical imaging units and the amount of absorbed light of the given frequency can be measured by the one or more optical imaging units. This technology is used in photoplethysmography (PPG), capnography and hemoencephalography (HEG). Far-infrared light is usually applied in therapeutic treatments rather than diagnostic procedures, but can also be incorporated in motion sensors.

The additional medical imaging units may comprise or be configured as one or more electrical impedance measurement units. The one or more electrical impedance measurement units may be configured as one or more electrical impedance measurement units with or without additional stimulation/transmission units. In this way, electrical stimulation measurements can be carried out. Electricity can be used to measure action potentials between different electrodes. This measurement is used in electrocardiography (ECG), nerve conduction studies (NCS), electroencephalography (EEG), electrodermography (EDG), electromyography (EMG) and rheoencephalography (REG). The method can be invasive where needles are inserted into the body or non-invasive where the integrated surface signal is measured (e.g. surface EMG or sEMG).

By combination of thermography with an additional diagnostic measurement, the limitation of just having a single information, i.e. the accurate body temperature, is lifted. An elevated temperature can be combined with results from additional diagnostic measurements such as ultrasound elastography. In this way, accurate and reliable image information is provided, while the medical imaging device used is portable.

Imaging resolution of specific medical indications can be increased by combining/joining ultrasound and thermography. For example, the limit on measuring muscle injuries can be lifted by increasing the information received by the one or more thermographic sensing units. The combination of ultrasound and thermography may be further refined or improved by additional information from yet another additional source such as one or more other additional medical imaging units as described herein. For example, to be able to resolve micro-cracks despite the lower resolution of ultrasound at larger penetration depths, an additional information from a different source is needed. Since micro-cracks go hand in hand with an inflammatory response in the body, which in turn surfaces as elevated temperature in the body, an integrated thermography can be pursued. In this way, with combined ultrasound and thermography, reliable medical image data can be provided with adequate resolution which allows to resolve micro-cracks. The additional information can, additionally or alternatively, come from a different measuring source, e.g. an optical measurement. Further, combined ultrasound and thermography may enable to distinguish between inflammation in muscle or other soft tissue, i.e. whether an inflammation is located in muscle or other soft tissue.

Muscle injuries may be visualized using the measurement data provided by the medical device, e.g. the portable medical (imaging) device. For this purpose, an algorithm may be designed that uses the measured data to reconstruct an image of the measured body part. For example, ultrasound (or other additional data) and thermographic data may be combined to analyze them for features and compare them with neighboring tissue. In this way, cracks in muscles, ligaments and tendons can be recognized and visualized. In addition, an error/accuracy estimation may be provided so that users/readers can estimate themselves how well the algorithm performs. In case of doubt, the user can move the medical device, e.g. the portable medical (imaging) device, a little and take a new "picture" (measurement) to allow for the algorithm to give more accurate results.

Diagnosis may be simplified through comparison. Once the reconstructed picture is in place the user might still not be sure what the data means. Therefore, the user may receive an idealized picture of the current body part in a healthy state and further an algorithm looks up a randomized, anonymous image made previously with another device, that shows the same body part in an unhealthy state. The images are looked up from the cloud, where all pictures that were taken are stored securely. The user can thereby make a more educated guess what the reconstructed image received from the measurement entails. The platform may contain a crowd-sourced element which means that it is possible to up-vote and down-vote diagnoses made by others and add new diagnosis suggestions. After a safe amount of statistics is gathered, the machine learning algorithm may be trained on the dataset and constant comparisons of the machine and human-made responses and suggestions can be made. Once the machine learning algorithm beats the human-made responses in accuracy, it may be used as default for the interpretation of a reconstructed image.

By way of the medical device, e.g. the portable medical (imaging) device injuries can be diagnosed faster and with less effort. Furthermore, the medical device, e.g. the portable medical (imaging) device may help to prevent accidents happening during physical exercise through a regular monitoring of the respective muscles, ligaments and tendons. This is of particular interest in the field of competitive sports. Thanks to the artificially intelligent evaluation and visualization software, the medical device, e.g. the portable medical (imaging) device cannot only be used by doctors, but also by physiotherapists and personal trainers with a medical background. This can be important for athletes since they spend more time with their physiotherapists and personal trainers than their doctors as long as they are not seriously sick.

The medical device, e.g. the portable medical (imaging) device, may also have one or more of the following advantages: The medical device, e.g. the portable medical (imaging) device, may be easy to clean. The medical device, e.g. the portable medical (imaging) device, may be flexible or may at least have a flexible measurement layer for contact with skin or the body surface. The at least one measurement layer may have more or less the size of a tablet. The medical device, e.g. the portable medical (imaging) device, may facilitate the highest possible accuracy, e.g. to resolve micro-cracks in muscles. Ultrasound gel might not be needed; instead silicone layers may be used in the medical device, e.g. the portable medical (imaging) device. Thermography and ultrasound may take their measurements at the same time to get two data points for one measured spot. This may allow for a proper evaluation and reduce measurement mistakes. The medical device, e.g. the portable medical (imaging) device may have limited weight so as to possibly use the device with one hand.

It is clear to a person skilled in the art that the statements set forth herein may be implemented under use of hardware circuits, software means, or a combination thereof. The software means can be related to programmed microprocessors or a general computer, an ASIC (Application Specific Integrated Circuit) and/or DSPs (Digital Signal Processors). For example, the processing layer and/or the one or more processing units may be implemented partially as a computer, a logical circuit, an FPGA (Field Programmable Gate Array), a processor (for example, a microprocessor, microcontroller (µC) or an array processor)/a core/a CPU (Central Processing Unit), an FPU (Floating Point Unit), NPU (Numeric Processing Unit), an ALU (Arithmetic Logical Unit), a Coprocessor (further microprocessor for supporting a main processor (CPU)), a GPU (Graphics Processing Unit), a GPGPU (General Purpose Computation on Graphics Processing Unit), a multi-core processor (for parallel computing, such as simultaneously performing arithmetic operations on multiple main processor(s) and/or graphical processor(s)) or a DSP. It is further clear to the person skilled in the art that even if the herein-described details will be described in terms of a method, these details may also be implemented or realized in a suitable device, a computer processor or a memory connected to a processor, wherein the memory can be provided with one or more programs that perform the method, when executed by the processor.

Even if some of the aspects described above have been described in reference to the medical device, e.g. the portable medical (imaging) device, these aspects may also apply to the medical system and the method and vice versa.

It is also to be understood that the terms used herein are for purpose of describing individual embodiments and are not intended to be limiting. Unless otherwise defined, all technical and scientific terms used herein have the meaning which corresponds to the general understanding of the skilled person in the relevant technical field of the present disclosure; they are to be understood too neither too far nor too narrow. If technical terms are used incorrectly in the present disclosure, and thus do not reflect the technical concept of the present disclosure, these should be replaced by technical terms which convey a correct understanding to the skilled person in the relevant technical field of the present disclosure. The general terms used herein are to be construed based on the definition in the lexicon or the context. A too narrow interpretation should be avoided.

It is to be understood that terms such as e.g. "comprising" "including" or "having" etc. mean the presence of the described features, numbers, operations, acts, components, parts, or combinations thereof, and do not exclude the presence or possible addition of one or more further features, numbers, operations, acts, components, parts or their combinations.

Although terms like "first" or "second" etc. may be used to describe different components or features, these components or features are not to be limited to these terms. With the above terms, only one component is to be distinguished from the other. For example, a first component may be referred to as a second component without departing from the scope of the present disclosure; and a second component may also be referred to as a first component. The term "and/or" includes both combinations of the plurality of related features, as well as any feature of that plurality of the described plurality of features.

In the present case, if a component is "connected to", "in communication with" or "accesses" another component, this may mean that it is directly connected to or directly accesses the other component; however, it should be noted that another component may be therebetween. If, on the other hand, a component is "directly connected" to another component or "directly accesses" the other component, it is to be understood that no further components are present therebetween.

In the following, specific embodiments of the present disclosure will be described with reference to the accompanying drawings; the same components are always provided with the same reference symbols.

In the description of the present disclosure, detailed explanations of known connected functions or constructions are omitted, insofar as they are unnecessarily distracting from the present disclosure; such functions and constructions are, however, understandable to the skilled person in the technical field of the present disclosure. The accompanying drawings are illustrative of the present disclosure and are not to be construed as a limitation. The technical idea of the present disclosure is to be construed as comprising, in addition to the accompanying drawings, all such modifications, variations and variants.

Other objects, features, advantages and applications will become apparent from the following description of non-limiting embodiments regarding the accompanying drawings. In the drawings, all described and/or illustrated features, alone or in any combination form the subject matter disclosed therein, irrespective of their grouping in the claims or their relations/references. The dimensions and proportions of components or parts shown in the figures are not necessarily to scale; these dimensions and proportions may differ from illustrations in the figures and implemented embodiments.
- Figure 1a: schematically shows a medical device according to a first embodiment;
- Figure 1b: schematically shows a medical device according to a second embodiment;
- Figure 2: schematically shows implementation details of a measuring layer of the medical device of figure 1a or figure 1b;
- Figure 3: schematically shows an output on a display related to the medical device of figure 1a or figure 1b;
- Figure 4a: schematically shows a medical device according to a first variant of the first embodiment or second embodiment; and
- Figure 4b: schematically shows a medical device according to a second variant of the first embodiment or second embodiment.

The variants of the functional and operational aspects as well as their functional and operational aspects described herein are only for a better understanding of its structure, its functions and properties; they do not limit the disclosure to the embodiments. The figures are partially schematic, said essential properties and effects are clearly shown enlarged or scaled down in part to clarify the functions, active principles, embodiments and technical characteristics. Every operation, every principle, every technical aspect and every feature that/which is disclosed in the figures or in the text is/can be combined with all claims, each feature in the text and the other figures, other modes of operation, principles, technical refinements and features that are included in this disclosure, or result from it, so that all possible combinations are assigned to the devices and methods described. They also include combinations of all individual comments in the text, that is, in each section of the description, in the claims and combinations between different variations in the text, in the claims and in the figures, and can be made to subject-matter of further claims.

The claims do not limit the disclosure and therefore the possible combinations of all identified characteristics among themselves. All features disclosed are explicitly also individually and in combination with all other features disclosed herein.

Accordingly, while further examples are capable of various modifications and alternative forms, some particular examples thereof are shown in the figures and will subsequently be described in detail. However, this detailed description does not limit further examples to the particular forms described. Further examples may cover all modifications, equivalents, and alternatives falling within the scope of the disclosure. Like numbers refer to like or similar elements throughout the description of the figures, which may be implemented identically or in modified form when compared to one another while providing for the same or a similar functionality.

In the following, without being restricted thereto, specific details are set forth to provide a thorough understanding of the present disclosure. However, it is clear to the skilled person that the present disclosure may be used in other embodiments, which may differ from the details set out below.

For example, while in the following the medical device is only described as portable medical imaging device 100 and is only referred to as portable medical imaging device 100, this only serves for explanation rather than limitation. In other words, the medical device described herein can be realised as being non-portable as well and can be realised with or without any display functionality.

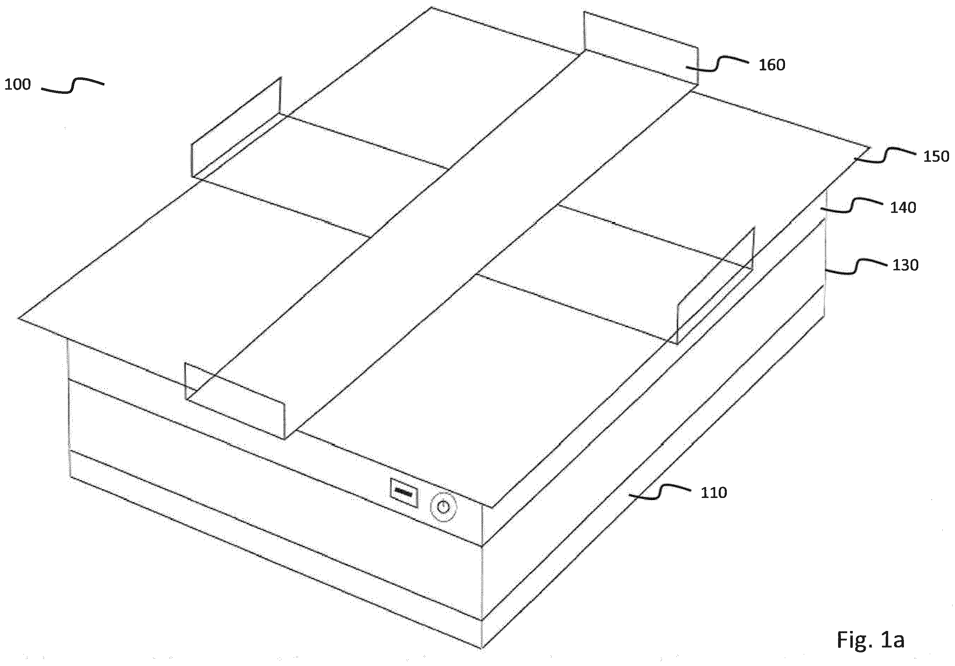

Figure 1a schematically shows a portable medical imaging device 100 according to a first embodiment.

The portable medical imaging device 100 comprises a measuring layer 110 and a processing layer 140. In addition, the portable medical imaging device 100 may comprise a buffer layer 130, a top portion 150 and a holding section 160.

As can be seen in figure 1a, the measuring layer 110 is arranged at the bottom of the portable medical imaging device 100 and thus can be referred to as bottom layer. The bottom of the portable medical imaging device 100 is the part of the device that is closest to the body of the patient or the probe when the portable medical imaging device 100 is used. The measuring layer 110 can be brought into close or in direct contact with the skin of a patient or client.

The processing layer 140 may be formed of solid material. The processing layer, in this example, comprises electronics, wiring, printed circuit boards (PCBs), processing units and the like.

The optional buffer layer 130 is arranged between the measuring layer 100 and the processing layer 140 and thus may be referred to as middle layer. The buffer layer aids in the measurement of how much the measuring layer 110 is bent/curved and where.

The optional top portion 150 of the portable medical imaging device 100 is of solid/robust material. The top portion 150 may be used for better handling the portable medical imaging device 100. The top of the device can have a holding section 160. The holding section 160 can be configured as or comprise one or more holding clips for holding a display device, a tablet or smartphone or the like.

The portable medical imaging device 100 may have a wireless connection, a USB port, an on/off switch and the like. The device may have the size of a tablet (around 10 inches) or a similar size.

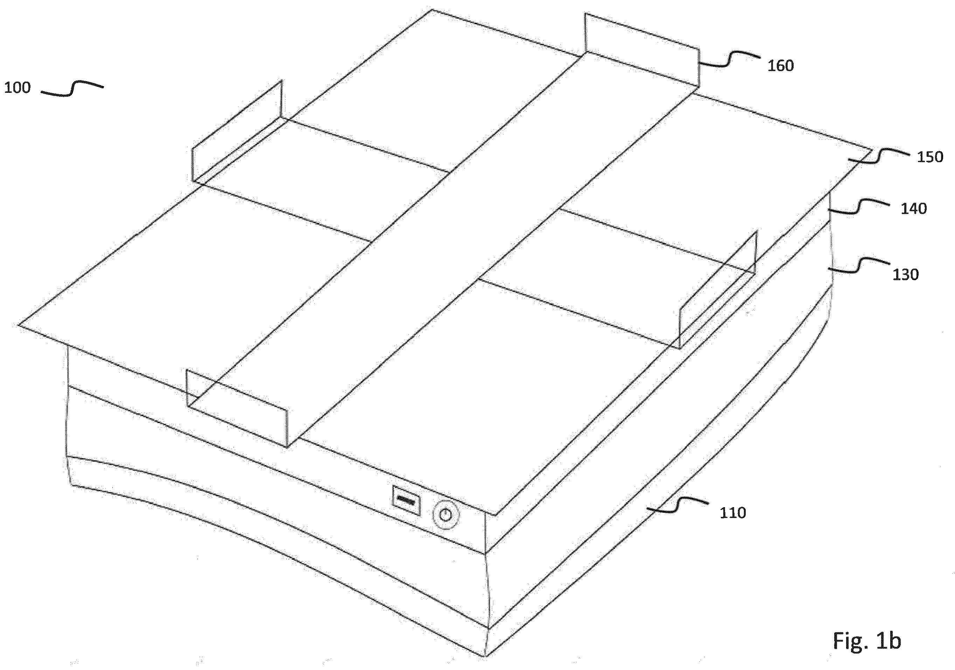

Figure 1b schematically shows a portable medical imaging device 100 according to a second embodiment. The portable medical imaging device 100 according to the second embodiment essentially corresponds to the portable medical imaging device 100 according to first embodiment but has a bendable measuring layer 110 and/or a bendable buffer layer 130. The embodiment shown in figure 1b has both a bendable measuring layer 110 and a bendable buffer layer 130, by way of explanation rather than limitation.

As the measuring layer 110 can be bent, it can maintain a substantially constant distance from the surface of the body along the lower/bottom surface of the measuring layer 110. For example, the measuring layer 110 can maintain its contact to the surface of the body. In this way, measurement accuracy is increased.

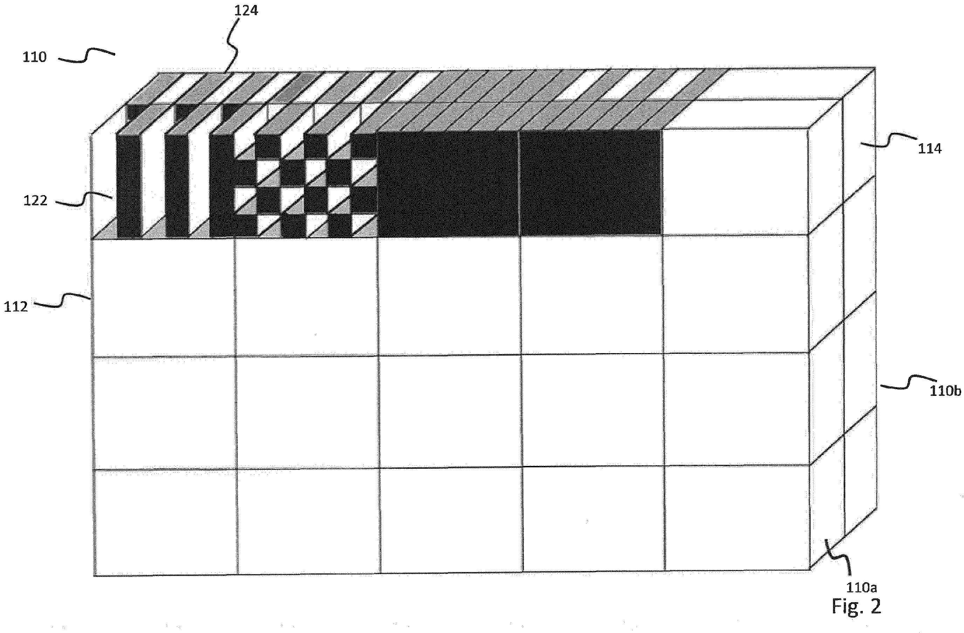

More details and aspects are mentioned in connection with the embodiments described above or below. The embodiments shown in figures 1a and 1b may comprise one or more optional additional features corresponding to one or more aspects mentioned in connection with the embodiment described below with reference to figure 2.

More particularly, in figure 2, the measuring layer 110 has (e.g. consists of), by way of example, a plurality of measuring layers which may be called sub-measuring layers 110a, 110b. The sub-measuring layers 110a, 110b are called first measuring layer 110a and second measuring layer 110b in the description of figure 2 as they can be considered full measuring layers regarding their functionality. The term sub-measuring layers may imply that both are part of one common measuring layer. Both measuring layers 110a, 110b are segmented in a plurality of segments 112, 114. In other words, each of the first measuring layer 110a and the second measuring layer 110b has a plurality of segments 112, 114. For sake of simplicity all segments of the first measuring layer 110a are referenced with one single reference sign 112. Similarly, all segments of the second measuring layer 110b are referenced with one single reference sign 114.

Figure 2 further shows examples for different arrangements of sensing units 122, 124 possible within one segment. Depending on the imaging technology used, the sensing units 122, 124 may be mere sensing units or be emitting/transmitting and sensing units. Although different patterns are used in each segment, the pattern in each segment of one or both measuring layers 110a, 110b may be the same.

As can be seen in figure 2, the measurement layer 110 has (e.g. consists of) a first measuring layer 110a and a second measuring layer 110b. The first measuring layer 110a has (e.g. consists of) different/multiple segments 112. The second measuring layer 11b has (e.g. consists of) different/multiple segments 114. Each measuring layer (sub-layer) 110a, 110b contains sensing (and optionally transmitting) units 122, 124 of a different technology. The sensing (and transmitting) units 122, 124 of the different technologies can overlap but do not have to overlap.

It is assumed by way of explanation rather than limitation that the first measuring layer 110a has thermography sensing units 122. In this case, the sensing units 122 do not include any kind of transmitting units. It is further assumed by way of explanation rather than limitation that the second measuring layer 110b has ultrasound sensing units 124. In this case, the sensing units 124 include ultrasound transmitting units and ultrasound sensing units and can be grouped together in sensing units 124 having a transmitting and sensing functionality. For sake of efficiency such sensing units 124 having a transmitting and sensing functionality will only be referred to as sensing units 124 in the following.

The segments 112, 114 can follow a checkerboard or striped pattern. A checkerboard pattern is shown, by way of example, in figure 2. The sensing units 122, 124 in the segments 112, 114 of the measuring layers 110a, 110b can have different patterns, i.e. the sensing units 122, 124 of the two measuring layers 110a, 110b can be arranged differently. The sensing units 122, 124 in the segments 112, 114 of the measuring layers 110a, 110b can have the same patterns, i.e. the sensing units 122, 124 of the two measuring layers 110a, 110b can be arranged in the same pattern. In one example, the pattern of the sensing units 122, 124 in each measuring layer 110a, 110b can be uniform across all segments 112, 114 but different or the same in the different measuring layers 110a, 110b.

Turning to the example of figure 2, in the first segment 112 (the left in the uppermost row of segments) of the first measuring layer 110a, three sensing units 122 are arranged with a gap between each other. In the first segment 114 (the left in the uppermost row of segments) of the second measuring layer 110b, three sensing units 124 are arranged with a gap between each other. The three sensing units 124 in the second measuring layer 114 do not overlap (in the height and width direction of the measuring layer 110) the three sensing units 122 in the first measuring layer 110a.

In the next segment 112 to the right of the first measuring layer 110a, twelve sensing units 122 are arranged with a gap between each other in a checkerboard pattern and oriented in a perpendicular direction. In the next segment 114 to the right of the second measuring layer 110b, twelve sensing units 124 are arranged with a gap between each other in a checkerboard pattern and oriented in a perpendicular direction. The sensing units 124 in the second measuring layer 110b do not overlap (in the height and width direction of the measuring layer 110) the sensing units 122 in the first measuring layer 110a. Alternatively, the segment 114 in the second measuring layer 110b may include multiple, e.g. three, stripe-shaped sensing units 124 similar to the corresponding segment 114 to the left. This facilitates a good or even ideal resolution, while noise tests can be performed. By way of the noise tests noise resulting from the sensing units 122 arranged in the first measuring layer 110a and overlapping the stripe-shaped sensing units 124 noise and distortions can be measured.

In the next segment 112 to the right of the first measuring layer 110a, six sensing units 122 are arranged without a gap between each other but directly adjacent to each other. In the next segment 114 to the right of the second measuring layer 110b, six sensing units 124 are arranged without a gap between each other but directly adjacent to each other. The sensing units 124 in the second measuring layer 110b overlap (in the height and width direction of the measuring layer 110) the sensing units 122 in the first measuring layer 110a. In other words, the sensing units 124 in the second measuring layer 114 follow the same pattern and are arranged at the same positions (in the height and width direction of the measuring layer 110) as the sensing units 122 in the first measuring layer 110a.

In the next segment 112 to the right of the first measuring layer 110a, six sensing units 122 are arranged without a gap between each other but directly adjacent to each other. In the next segment 114 to the right of the second measuring layer 110b, three sensing units 124 are arranged with a gap between each other. The sensing units 124 in the second measuring layer 110b partially overlap (in the height and width direction of the measuring layer 110) the sensing units 122 in the first measuring layer 110a. In other words, a subset (three in the present example) of the sensing units 122 in the first measuring layer 110a overlaps (in the height and width direction of the measuring layer 110) with the sensing units 124 in the second measuring layer 110b. Another subset (three in the present example) of the sensing units 122 in the first measuring layer 110a does not overlap (in the height and width direction of the measuring layer 110) with the sensing units 124 in the second measuring layer 110b.

Any of the numbers and arrangements described with respect to figure 2 are purely exemplary and can be adapted according to the specific requirements needed.

The segments 122, 124 may be connected in such a way that they keep the overall measuring layer 110 flexible (for being bent). At least parts of the measuring layers can be movable. In the example shown in figure 2, the complete first measuring layer 110a may be movable. When moving the first measuring layer 110a the second measuring layer 110b can carry out measurements without any distortions or influences potentially caused by the first measuring layer 110a. In this way, measurement accuracy can be improved. Alternatively, only portions, e.g. one or more segments 112, of the first measuring layer 110a may be movable.

Turning to the specific example shown in figure 2, it might not be necessary to move the first segment 112 (the left in the uppermost row of segments) and the next segment to the right in the first measuring layer 110a. This is because the sensing units 122 in the first measuring layer 112 and the sensing units 124 in the second measuring layer 110b do not overlap and so influences and distortions between the sensing units 122, 124 are reduced. However, it might be beneficial to move the other two segments 112 in the first measuring layer 110a as the sensing units 122, 124 in the respective segments 112, 114 of the first and second measuring layers 110a, 110b at least partially overlap each other and thereby might cause some distortions influencing measurement accuracy.

The measuring layers 110a, 110b can either be wrapped into a third material in such a way that they appear to belong to a single measuring layer 110 from the outside. Or an additional layer of a third material is attached to the measuring layers 110a, 110b and can be disposed of after each or several uses of the portable medical imaging device 100. The information coming from the measurement layer 110 can be partially processed within the portable medical imaging device 100 and/or partially processed by connecting to a server/cloud solution.

The one or more sensing units 122 in the first measuring layer 110a may be one or more thermographic sensing units. The one or more sensing units 124 in the second measuring layer 110b may one or more ultrasound imaging units. Alternatively, the one or more sensing units 124 in the second measuring layer 110b may be at least one of one or more thermoacoustic imaging (TAT) units, one or more x-ray or gamma-ray radiography imaging units, one or more magnetometric imaging units, one or more elastographic imaging units, one or more ultrasound elastography imaging units, one or more electrographic imaging units, one or more photoacustic/optoacoustic imaging (PAT) units, one or more magnetometric imaging units, one or more electrical impedance measurement (with or without additional transmitting/external stimulation units) imaging units, one or more magnetic resonance imaging units, one or more functional magnetic resonance imaging units, one or more tactile imaging/mechanical imaging units, one or more medical optical imaging units.

By way of example, the portable medical imaging device 100 as described with respect to figures 1a to 2 can be used for the following areas of application.

For example, inflammations can be determined in a precise manner. In general, inflammations follow the following five indications, namely increased temperature, pain, redness, swelling, and loss of function. In order to detect inflammations, the one or more thermographic sensing units 122 can detect an elevated temperature with respect to the surrounding. This detection is usually a 2D measurement.

The one or more ultrasound sensing units 124 are used to exclude that other factors rather than inflammation are the actual cause of the elevated temperature. For this purpose, the one or more ultrasound sensing units 124 may be controlled by one or more control units in the processing layer 140, to carry out ultrasound measurements. The one or more ultrasound sensing units 124 may be controlled by one or more control units to carry out ultrasound measurements at essentially the same point of time or period of time at/during which the one or more thermographic sensing units 122 carry out thermographic measurements. For example, the one or more ultrasound sensing units 124 may be configured to carry out ultrasound elastography to infer a swelling of the portion/probe under examination. Elevated temperature (as detected by thermography) plus swelling (as detected by ultrasound elastography) can be a clear sign for inflammation.

To finally verify the diagnosis of inflammation, invasive blood tests may be performed.

As another example, muscle and/or tendon and/or ligament injuries can be detected. The injuries of muscles, tendons and/or ligaments can have different sizes. There can be complete tears, partial tears (macroscopic cracks) or single tears (microscopic cracks, at the level of a single muscle fibril). The injuries can be seen with ultrasound up to a certain resolution. If they are too small (below ∼1 cm crack length), standard ultrasonic measurements do not allow for a measurement that is accurate enough.

As the resolution of ultrasound measurements is limited, in particular in deeper muscle tissue, ultrasound measurements may be replaced by or combined with MRI. MRI provides perfect resolution. However, MRI is not only costly but also requires much time to get the required information. In addition, MRI cannot be integrated into a portable device.

Since micro-cracks go hand in hand with an inflammatory response in the body, which in turn surfaces as elevated temperature in the body, an elevated temperature as measured by the one or more thermographic sensing units 122 can be combined with ultrasound measurements of the one or more ultrasound sensing units 124. In this way, with combined ultrasound and thermography, reliable medical image data can be provided with adequate resolution which allows to resolve micro-cracks. The additional information can additionally or alternatively, come from a different measuring source, e.g. an optical measurement.

As still another example, other myographic characteristics can be detected. While invasive electromyography is known as the gold standard and to be highly accurate in giving a three-dimensional image of the muscle strength, surface myography has been shown to give very good results as well, even though it is limited to two dimensions. A myography can be performed with ultrasound or electric currents. More particularly, a myography can be performed with electrodiagnostic medicine technique, nerve conduction tests, muscle and nerve biopsy, muscle enzymes, serologic studies, ultrasound and MRI. In conjunction with the thermographic sensing units 122, the source of an inflammation can be found. In particular, inflammations within muscles can be distinguished from ligament, tendon or other soft tissue inflammations. Furthermore, the absence of an elevated temperature allows to identify for example a reduced nerve activity, muscle atrophy or other muscular and non-muscular illnesses which do not go hand in hand with an inflammation. Furthermore, trigger points can be located in places of elevated temperature and tissue stiffness, and the degree of a muscle's tenseness can be gauged. The monitoring of changes in temperature of a body part furthermore allow to infer sources of inflammations (autoimmune vs. infectious disease).

For this purpose, the one or more ultrasound sensing units 124 may carry out ultrasound measurements. The measurements make it possible to derive myographic characteristics. For example, the one or more ultrasound sensing units 124 may be configured to carry out ultrasound elastography to detect muscle atrophy or muscle stiffness (= muscle spasms, tenseness, hypertension and hypotension).

Myography can give information on the strength of the muscle or detect muscle shrinkages (muscle atrophy) over time or in comparison with neighboring muscles or the symmetric partner muscle (e.g. right arm muscle compared to left arm muscle). It can further measure muscle spasms, tenseness, hypertension and hypotension. Further, it can be desirable to measure the signal transmitted by the brain into the muscle to detect neurological problems.

The obtained information can be refined by carrying out electrical stimulation of a portion or a probe under examination, in particular the muscle. In this way, it is possible to measure muscle strength directly and during movement (e.g. while performing sports) or to measure nerve functioning. The obtained information can be refined by using laser diode light. Laser diode light can be used to measure muscle tension, for example. The obtained information can be refined by using highly accurate pressure sensors. Such pressure sensors may be used to measure muscle tension, for example. The obtained information can be refined by using magnetometry. Magnetometry may be used to determine nerve location and measure nerve functioning. Previously, magnetometry was only used in non-portable devices or needs to be extremely cooled (e.g. to be able to use superconducting quantum interference devices (SQUIDS)).

Bones are normally located by way of x-ray based devices such as CT devices. The location of bones is interesting for the detection of bone deformations (for example Scoliosis), fractures and fissures. Ultrasound can be used for this purpose but usually provides very limited resolution. Maximally a bone deformation can be detected if the ultrasound frequency is low enough but then the resolution is also very low). As still another example that can be detected by way of the portable medical imaging device 100, it becomes possible to infer information on bone deformations if these appear together with overly tense or shortened muscles or muscle calcifications or ossifications. One example could be the detection of the Osgood-Schlatter disease. Here, the thermographic image would show a higher and/or lower than expected temperature in the respective areas.

To refine the obtained information CT or x-ray (radiographic imaging) in general can be used. To refine the obtained information MRI can be used. To refine the obtained information magnetometry can be used. Magnetometry can be used to deduce bone locations.

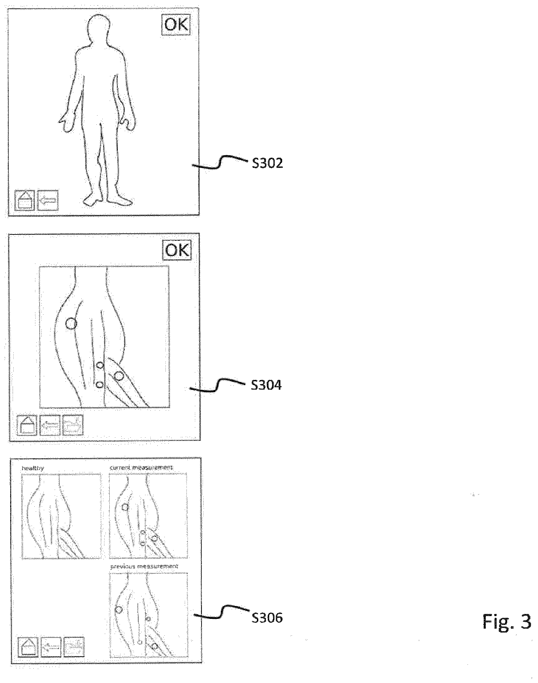

Data analysis and evaluation is described with respect to figure 3. The algorithm has two main ingredients: 1) an image reconstruction of the measured detail of the body and 2) the comparative analysis of the measurement with other measurements stored in the (company) cloud.

To allow for a reconstruction and comparison of the image, on a first display the user first chooses the rough location the measurement should be performed at. This is allowed by offering the user a schematic image of a human body (can be enhanced by additional texts and pop-ups) to choose from (see step S302). The image does not have to be limited to human bodies. Animals and objects can also be depicted and targeted as measurement subject (top-most image).

After having chosen the rough location of measurement, the measurement is performed and the reconstructed image is visualized on the second display (in real-time if possible) (see step S304). Internally, the algorithm converts the measured data received into an image that can but does not have to have similarities with illustrations broadly known from anatomy atlases. It detects anomalies by comparing data with the nearest-neighbor-measurements and visualizes these by circled spots. The decision of circling or not circling is made based on the computed accuracy of the algorithm, which in turn is determined by using the appropriate statistical tools. When clicking on each spot, the accuracy of the algorithm is quoted, such that the user can develop an intuition for the interpretation of the result.

The measured image can be saved to the cloud or exported into different formats.

To allow users to make an even more informed diagnosis, the user can enter a third display (see step S306). In the third display, the user receives other comparative pictures. The first picture is an illustration of the same test area (decided upon in the first display) shown in a completely healthy state. The second picture is the measurement just taken. The third picture is a comparison of the test area with previous pictures of this area. Internally, the algorithm extracts the features of the measurement image and looks up similarities with previous measurement images that have shown similar circled areas. Along with the third image, additional information is shipped. This comprises of how others have diagnosed this measurement, how often the diagnoses were made and how many people were unsure about a diagnosis. With this information the user has full access to the algorithmically derived results. With an increase in measurements taken, a machine learning algorithm is set to propose own diagnoses. These are put in comparison/competition with the results derived by humans and serve as additional diagnoses suggestions. The three comparison pictures/sub-displays can be augmented by further sub-displays and/or exchanged.

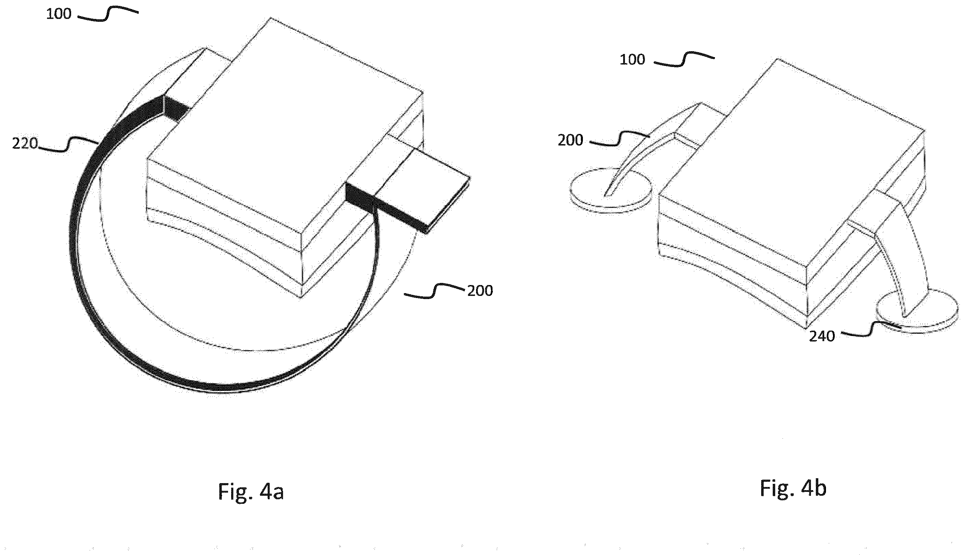

Figure 4a schematically shows a medical device according to a first variant of the first embodiment of figure 1a or the second embodiment of figure 1b. Figure 4b schematically shows a medical device according to a second variant of the first embodiment of figure 1a or the second embodiment of figure 1b. More specifically, figures 4a and 4b show examples for mounting units that can be realised on the portable medical imaging device 100 according to the first embodiment of figure 1a or according to the second embodiment of figure 1b. In figure 1a, the mounting unit 200 includes or is configured as a combination of straps 220. The straps 220 may comprise or be made of an easy-to-clean material, e.g. an elastic rubber-based band. The straps 220 may, when mounted, enclose the portion of the body or body part in question. In figure 4b, the mounting unit 200 includes or is configured as a combination of temporarily-adhesive foam cushions 240. These cushions 240 can be attached to the portion of the body or the body part in question to mount the portable medical imaging device 100 to the said portion of the body or body part in question.

The aspects and features mentioned and described together with one or more of the previously detailed examples and figures, may as well be combined with one or more of the other examples in order to replace a respective feature of the other example or in order to additionally introduce the feature to the other example.

Examples may further be or relate to a computer program having a program code for performing one or more of the above methods, when the computer program is executed on a computer or processor. Steps, operations or processes of various above-described methods may be performed by programmed computers or processors.

Examples may also cover program storage devices such as digital data storage media, which are machine, processor or computer readable and encode machine-executable, processor-executable or computer-executable programs of instructions. The instructions perform or cause performing some or all of the acts of the above-described methods. The program storage devices may comprise or be, for instance, digital memories, magnetic storage media such as magnetic disks and magnetic tapes, hard drives, or optically readable digital data storage media.

Further examples may also cover computers, processors or control units programmed to perform the acts of the above-described methods or (field) programmable logic arrays ((F)PLAs) or (field) programmable gate arrays ((F)PGAs), programmed to perform the acts of the above-described methods.

The description and drawings merely illustrate the principles of the disclosure. Furthermore, all examples recited herein are principally intended expressly to be only for pedagogical purposes to aid the reader in understanding the principles of the disclosure and the concepts contributed by the inventor to furthering the art. All statements herein reciting principles, aspects, and examples of the disclosure, as well as specific examples thereof, are intended to encompass equivalents thereof.

A block diagram may, for instance, illustrate a high-level circuit diagram implementing the principles of the disclosure. Similarly, a flow chart, a flow diagram, a state transition diagram, a pseudo code, and the like may represent various processes, operations or steps, which may, for instance, be substantially represented in computer readable medium and so executed by a computer or processor, whether or not such computer or processor is explicitly shown. Methods disclosed in the specification or in the claims may be implemented by a device having means for performing each of the respective acts of these methods.

It is to be understood that the disclosure of multiple acts, processes, operations, steps or functions disclosed in the specification or claims may not be construed as to be within the specific order, unless explicitly or implicitly stated otherwise, for instance for technical reasons. Therefore, the disclosure of multiple acts or functions will not limit these to a particular order unless such acts or functions are not interchangeable for technical reasons. Furthermore, in some examples a single act, function, process, operation or step may include or may be broken into multiple sub-acts, -functions, -processes, -operations or -steps, respectively. Such sub acts may be included and part of the disclosure of this single act unless explicitly excluded.

Furthermore, the following claims are hereby incorporated into the description, where each claim may stand on its own as a separate example. While each claim may stand on its own as a separate example, it is to be noted that - although a dependent claim may refer in the claims to a specific combination with one or more other claims - other examples may also include a combination of the dependent claim with the subject matter of each other dependent or independent claim. Such combinations are explicitly proposed herein unless it is stated that a specific combination is not intended. Furthermore, it is intended to include also features of a claim to any other independent claim even if this claim is not directly made dependent to the independent claim.

The present disclosure is not limited in any way to the embodiments described above. On the contrary, there are many possibilities for modifications thereof, which are apparent to an average skilled person without departing from the underlying idea of the present disclosure as defined in the appended claims.

## Claims

1. A medical device comprising:
- at least one measuring layer, the at least one measuring layer comprising:
∘ one or more thermographic sensing units configured to detect infrared radiation emitted by a portion of a body of a user, and
∘ one or more additional medical imaging units configured to acquire additional imaging information about the portion of the body;
- a processing layer comprising one or more processing units configured to process the detected infrared radiation and the acquired additional imaging information to enable reconstruction of a combined medical image based on the detected infrared radiation and the acquired additional imaging information.

2. The medical device of claim 1, wherein the one or more thermographic sensing units are arranged in a first plane in a depth direction of the medical device, and the one or more additional medical imaging units are arranged in a second plane in the depth direction of the medical device, wherein the first plane is the same as the second plane or different from the second plane.

3. The medical device of claim 1 or 2, wherein the at least one measuring layer is configured as multiple measuring layers, wherein the one or more thermographic sensing units are arranged in a first measuring layer of the multiple measuring layers and the one or more additional medical imaging units are arranged in a second measuring layer of the multiple measuring layers, wherein the one or more thermographic sensing units arranged in the first measuring layer are arranged to overlap or to not overlap the one or more additional medical imaging units arranged in the second measuring layer.

4. The medical device any one of the previous claims, wherein at least one or more portions of the at least one measuring layer are bendable and/or movable.

5. The medical device of any one of the previous claims, wherein the at least one measuring layer comprises a plurality of measuring sections, wherein the measuring sections are respectively separated from each other by one or more slits or recesses.

6. The medical device of any one of the previous claims, wherein the processing layer further comprises one or more control units configured to control the one or more thermographic sensing units to detect the infrared radiation at a first period of time and to control the one or more additional medical imaging units to acquire the additional imaging information at a second period of time, wherein the first period of time and the second period of time at least partly overlap each other, for example substantially coincide with each other.

7. The medical device of any one of the previous claims, the medical device further comprising a buffer layer between the at least one measuring layer and the processing layer.

8. The medical device of claim 7, wherein the buffer layer is bendable.

9. The medical device of any one of the previous claims, the medical device further comprising a mounting unit configured to mount the medical device to the body.

10. The medical device of any one of the previous claims, the medical device further comprising a holding section configured to hold a display device.

11. The medical device of any one of the previous claims, wherein the medical device is a portable medical imaging device and/or the medical device further comprises a communication interface for communicating with one or more server units.

12. The medical device of any one of the previous claims, wherein the one or more processing units are configured to
- reconstruct the combined medical image based on the detected infrared radiation and the acquired additional imaging information; and/or
- analyse the reconstructed combined medical image for elevated temperature, inflammations, injuries and other abnormalities or diseases related to inflammation in the portion of the body.

13. The medical device of any one of the previous claims, wherein the one or more additional medical imaging units comprise or are configured as at least one of one or more ultrasound imaging units, one or more thermoacoustic imaging, TAT, units, one or more x-ray or gamma-ray radiography imaging units, one or more magnetometric imaging units, one or more elastographic imaging units, one or more ultrasound elastography imaging units, one or more electrographic imaging units, one or more photoacustic/optoacoustic imaging, PAT, units, one or more magnetometric imaging units, one or more electrical impedance measurement (with or without additional transmitting/external stimulation units) imaging units, one or more magnetic resonance imaging units, one or more functional magnetic resonance imaging units, one or more tactile imaging/mechanical imaging units and one or more medical optical imaging units.

14. A medical system comprising
- the medical device of any one of claims 1 to 13; and
- one or more server units, wherein the one or more server units are configured to:
∘ reconstruct the combined medical image based on the detected infrared radiation and the acquired additional imaging information; and/or
∘ analyse the reconstructed combined medical image for elevated temperature, inflammations, injuries and other abnormalities or diseases related to inflammation in the portion of the body; and/or
∘ store a plurality of images of various portions of the body in healthy and unhealthy states; and/or
∘ provide one or more of a plurality of stored images of the portion of the body to the portable imaging device.

15. A method for enabling reconstruction of a combined medical image of a portion of a body of a user, the method comprising:
- detecting infrared radiation emitted by the portion of the body;
- acquiring additional imaging information about the portion of the body; and
- processing the detected infrared radiation and the acquired additional imaging information to enable reconstruction of the combined medical image based on the processed detected infrared radiation and the processed acquired additional imaging information.
